# EUROPEAN PATENT SPECIFICATION

(11) **EP 1 933 783 B1**
(45) Date of publication and mention of the grant of the patent: **15.09.2010**
(21) Application number: 06794757.2
(22) Date of filing: 13.10.2006
(51) Int. Cl.: A61F 5/455

(54) **URINE COLLECTION DEVICE**
URINSAMMELVORRICHTUNG
DISPOSITIF DE RECUEIL D'URINE

(30) Priority: 13.10.2005 GB 0520864
(43) Date of publication of application: 25.06.2008
(73) Proprietor: Brunel University, Middlesex UB8 3PH (GB); Loughborough University, Leicestershire LE11 3TU (GB)
(72) Inventor: VAN DEN HEUVEL, Eleanor, Slough SL1 7BD (GB); JOWITT, Felicity, Middlesex TW15 1LS (GB); O'CONNOR, Tara, Worcestershire DY9 9LH (GB)
(74) Representative: Jehan, Robert
(86) International application number: PCT/GB2006/003810
(87) International publication number: WO 2007/042823

(56) References cited:
- WO-A-00/57784
- WO-A-90/00379
- US-A1- 2005 137 557

## Description

The present invention relates to a pad for use with a urine collection device.

Millions of people world-wide are afflicted with the problem of urinary incontinence, the economic costs of this distressing condition are extremely high but the cost in terms of human suffering is incalculable. Both men and women are afflicted by the condition although many more women are affected and incidence increases with age.

For many younger men external catheter systems provide an adequate solution to the problem of severe urinary incontinence, but these systems are far less satisfactory for older men who are more likely to experience continence problems. For women continence management solutions are extremely limited. Internal catheters are associated with a host of medical problems, particularly urinary tract infections and urethral erosions. Large disposable pads to absorb urine are a safer option but are far from an ideal solution. Pads are bulky and require frequent changing if the wearer is to remain comfortable and free from odour problems. For immobile patients and those with sensory loss, wet pads can exacerbate the risk of pressure injury.

One of the maj or problems in designing a device to cope with incontinence is the very high flow rate which is a characteristic of urine flow. During a void the urine flow rate increases rapidly to a peak rate of 25-30 millilitres per second. To pump urine directly requires a powerful pump able to reach a high flow rate as soon as it is activated. This is extremely difficult to achieve and would require a much larger pump and power source than can be easily transported.

WO 00/57784 discloses a urine collection device in which a vacuum is maintained. When urine is detected at an interface between the device and its user, a valve is opened allowing the pumping of urine into a storage container. In this device, a layered interface between the device and its user is provided. The interface preferably comprises an outer layer that is impervious to air and liquids, above which is a spacing layer through which liquid can flow. A nylon mesh layer is provided through which the urine must flow before entering the urine outlet tube. A layer of shape-retaining material is provided to give structural stability to the interface, and to allow urine to flow along the length of the interface into a reservoir section. Finally, an upper receiving layer takes up urine and allows it to flow through to the lower layers.

The present invention seeks to provide an improved interface that may be used with the device disclosed in WO 00/57784. In particular, the improved interface seeks to reduce leakage that could result from the use of several different layers, which can slow the passage of urine through the interface towards the urine outlet tube.

According to an aspect of the present invention there is provided a pad assembly for use as a non-invasive interface of a urine collection device, comprising:
a liquid-impermeable external layer including a urine-receiving aperture, a upper end and a lower end);
a layer of flexible filling material through which urine is able to flow substantially freely, the filling material being partially surrounded by the external layer; and
an outlet tube for use in the withdrawal of urine from the pad assembly, the outlet tube extending through the external layer to the lower end of the pad assembly.

A pad assembly having these features allows urine to flow through without substantial build up within the pad assembly. It enables collection of urine from a user for delivery to a urine collection device whilst reducing problems of leakage.

Preferably the filling material is not multi-layer. In the preferred embodiment the filling material comprises a single layer. Provision of several different layers could slow the passage of urine through the interface and may result in leakage.

The filling material preferably allows urine to flow through at a rate of at least 25ml per second, more preferably at a rate of 30ml per second, even more preferably at a rate of at least 35ml per second. Urine flow rate can increase to a peak of 25-30ml per second. Such filling material therefore allows urine to flow through without substantially restricting its flow.

In the preferred embodiment, the filling material comprises an open, low density material. Such material is suitable and allows urine to flow through substantially freely.

Preferably, the filling material has a density of 0.03-0.01 g/cm³. This is suitable for a substantially free flow of urine.

The porosity of the filling material is preferably between 50% and 99% of its volume, more preferably between 60% and 99%, more preferably between 70% and 99%, more preferably between 75% and 99%, more preferably between 80% and 99%, more preferably between 85% and 99%, more preferably between 90% and 99%, and even more preferably between 95% and 99%. These porosities facilitate an unrestricted flow of urine.

The filling material may comprise a reticulated foam, a non-woven plastic fibre or a woven spacer fabric. These have been identified as suitable materials. Thus, in the preferred embodiment, urine is able to flow substantially freely through the filling material. This allows urine to flow through without substantial build up within the pad assembly. It enables collection of urine from a user for delivery to a urine collection device whilst reducing problems of leakage.

In the preferred embodiment, the interior of the pad assembly is able to accommodate an end portion of an urine outlet tube such that, in use, at least a portion of a urine outlet tube is accommodated longitudinally within the boundary of the exterior. This provides for a more compact arrangement, which helps maintain the urine outlet tube in position. It also serves to reduce leakage problems.

One end of the pad may be narrower than its opposite end. This facilitates channeling of urine to the urine outlet tube.

The urine outlet tube could be accommodated within the filling material, or between the filling material and the exterior. Whilst the second arrangement may be simpler, the first arrangement can result in a tube that is not visible along the lower part of the pad assembly.

In an embodiment, the pad assembly is able to accommodate the urine outlet tube along its full length. This enables the inlet to the outlet tube to be positioned at the lowest part (in use) of the pad assembly, which aids in urine collection.

Preferably a first lower, end of the pad assembly is narrower than its opposite higher, end. This facilitates channeling of urine to the urine outlet tube.

For instance, the pad assembly may include a taper at its lower, narrow end. This further facilitates urine channeling to the outlet tube.

The lower, narrow end of the pad assembly is preferably blunt and is preferably substantially the same size as the diameter of a urine outlet tube. This provides a suitable location for a urine outlet tube and helps to prevent urine leakage.

Preferably, a higher end of the pad is rounded. This provides comfort for the user.

Preferably the exterior of the pad assembly is conformable. This makes the pad more comfortable for the user.

The pad assembly preferably has a capacity of less than 200ml. A small pad assembly is less intrusive for the user.

The present invention also provides a urine collection device including a pad assembly as specified herein.

Preferred embodiments of the present invention are described below, by way of example only, and with reference to the accompanying drawings, in which:
Figure 1 shows an isometric view of a pad according to an embodiment of the invention;
Figure 2 shows a top view of the pad of Figure 1;
Figure 3 shows a longitudinal cross section along the line A-A of Figure 2; and
Figure 4 shows a longitudinal cross section along line B-B on Figure 2.

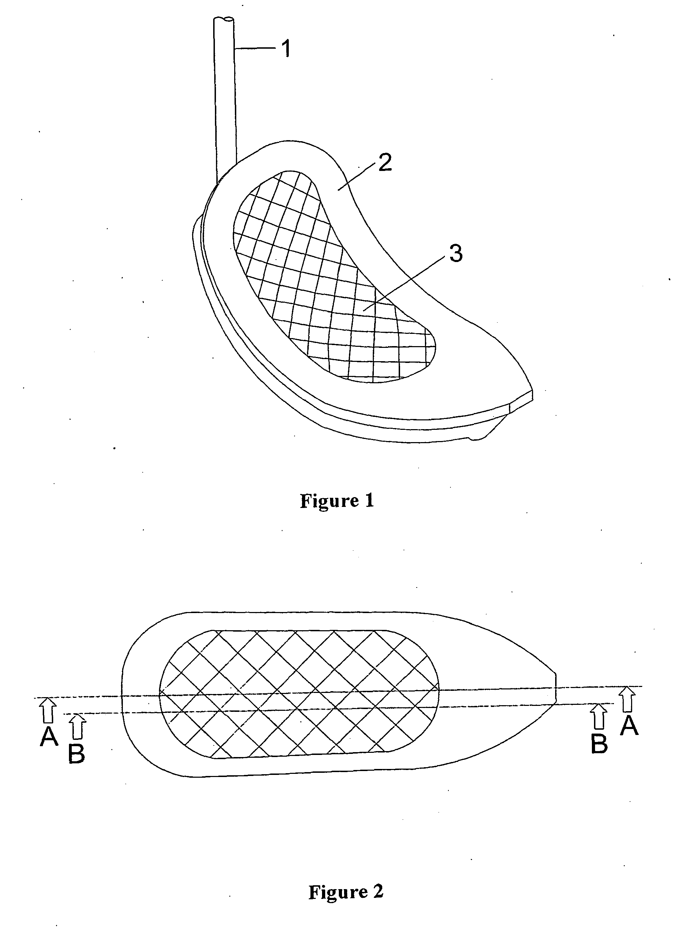

Referring to Figures 1 and 2, a preferred pad comprises an external layer 2 that partially surrounds filling material 3. The filling material 3 comprises a single layer of open low-density material that does not appreciably resist the flow of urine nor retain urine, and thereby allows urine to pass rapidly through. An example of a suitable material would be one that is non-woven, made up of fibres with open spaces between, such as the material used in scouring pads. The fibres may be tangled or may be held together with glue or resin. A suitable filling material may be described as a non-woven material made from tangled polymeric fibre to give a disorganised open web structure. The filling material 3 should be flexible and comfortable to sit on whilst retaining the open space within it.

The filling material 3 resists crushing but is still comfortable to wear. The density of the filling material 3 in this embodiment is 0.03-0.1g/cm³. The porosity of the filling material 3 is in this embodiment between 50% and 99% of the volume of the pad. Water should be able to enter the material at a flow rate of at least 35ml per second without surface run off and be able to run freely though the material towards a urine outlet tube.

The external layer 2 is an impermeable conformable material such as flexible plastic or expanded polystyrene. The external layer 2 should be reasonably soft, but able to retain its shape. It covers the filling material 3 apart from a rounded, rectangular hole in a position to correspond with the urethral opening of a user.

As shown in Figures 1 and 2, the pad has an upper rounded end (to be worn towards the front by a user), and a lower tapered end (to be worn towards the back by a user). This shape provides a more comfortable fit for the user, and helps to prevent leakage. It also aids channelling of urine to the urine outlet tube.

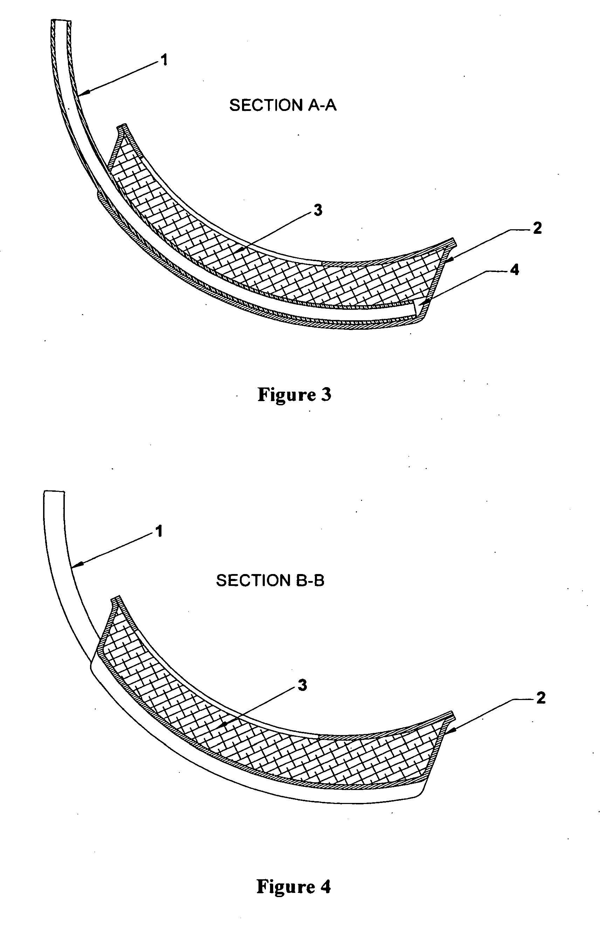

Figures 3 and 4 illustrate the arrangement of the filling material 3 within the external layer 2. The urine outlet tube 1 enters the pad at its upper (front) end and extends longitudinally along the full length of the pad, underneath the filling material 3, but inside the external layer 2. The open end of the urine outlet tube 1 fits within the tapered end 4 of the pad. In this embodiment, the urine outlet tube 1 has been accommodated by shaping the external impervious layer 2 around the outside of the tube.

The user places the pad inside their underwear. Specially adapted underwear including a mesh overlying the gusset could be used. The mesh serves to hold the pad in place.

Urine is received by the filling material 3 exposed through the hole in the external layer 2. Since the urine is able to flow freely through the filling material 3, gravity draws it towards the tapered end 4 of the pad and leakage is avoided. The end of the urine outlet tube 1 is located at the tapered end 4 of the pad. Urine is thus channelled towards the tapered end 4 of the pad from where it can be removed by the urine outlet tube 1.

There are several advantages of the above-described arrangement.

The single layer of filling material 3 ensures a rapid flow of urine through the pad and to the outlet tube, thereby minimising the possibility of leakage.

The location of the urine outlet tube 1 inside the external layer 2 ensures a good connection between the urine outlet tube 1 and the pad, which also helps to reduce the likelihood of leakage. The need for a specific connector between the urine outlet tube 1 and the pad is avoided. This arrangement is cheaper to produce than that previously described.

The rounded, upper end of the pad provides a comfortable fit, whilst the tapered, lower end 4 facilitates channelling of the urine to the urine outlet tube 1.

Various modifications can be made to the above-described arrangement.

The filling material 3 can be any suitable material that gives no effective resistance to a flow of urine. In other words, the urine should pass through the filling material as though it were not present. In particular, suitable materials for the filling material 3 include a reticulated foam, a non-woven plastic fibre, a woven spacer fabric or any similar low-density material.

The external layer 2 can be made from any suitable material having the desired properties. Suitable materials are described in WO 00/57784. Other suitable materials for the external layer 2 include impermeable materials such as a thin, soft closed cell foam or a closed cell polyethylene substance such as Plastazote® (a closed cell cross-linked polyethylene foam).

The hole in the external layer 2 through which urine is able to pass need not be rectangular. It could, for example, be oval, or any other suitable shape.

If the filling material 3 is a substance able to hold its shape well, the external layer 2 need not be anything more substantial than a simple impermeable cover.

Other arrangements for the urine outlet tube 1 within the pad can be envisaged. For example, the tube could be accommodated within the filling material 3 so that it is not visible along the lower part of the pad.

The skilled person will appreciate that the various modifications can be substituted or combined as appropriate.

## Claims

1. A pad assembly for use as a non-invasive interface of a urine collection device, comprising:
a liquid-impermeable external layer (2) including a urine-receiving aperture, an upper end and a lower end (4);
a layer of flexible filling material (3) through which urine is able to flow substantially freely, the filling material being partially surrounded by the external layer; and
an outlet tube (1) for use in the withdrawal of urine from the pad assembly, **characterized in** the outlet tube (1) extending through the external layer (2) to the lower end (4) of the pad assembly.

2. A pad assembly as claimed in claim 1, wherein the filling material (3) comprises a single layer.

3. A pad assembly as claimed in claim 1 or 2, wherein the filling material (3) allows urine to flow through at a rate of at least 25 ml/second.

4. A pad assembly as claimed in claim 3, wherein the filling material (3) allows urine to flow through at a rate of at least 35 ml/second.

5. A pad assembly as claimed in any preceding claim, wherein the filling material (3) comprises an open, low-density material.

6. A pad assembly as claimed in any preceding claim, wherein the filling material (3) has a density of 0.03-0.1g/cm³.

7. A pad assembly as claimed in any preceding claim, wherein the porosity of the filling material (3) is between 50% and 99% of its volume.

8. A pad assembly as claimed in any preceding claim, wherein the filling material (3) comprises reticulated foam, a non-woven plastic fibre or a woven spacer fabric.

9. A pad assembly as claimed in any preceding claim, wherein the pad assembly is able to accommodate a urine outlet tube (1) within the filling material (3).

10. A pad assembly as claimed in any preceding claim, wherein the interior of the pad is able to accommodate an end portion of a urine outlet tube (1) such that, in use, at least a portion of a urine outlet tube is accommodated longitudinally within the boundary of the exterior.

11. A pad assembly as claimed in any preceding claim, wherein the lower end (4) of the pad assembly is narrower than its upper end.

12. A pad assembly according to any preceding claim, wherein the lower end of the pad assembly is blunt and substantially the same size as the diameter of the outlet tube (1).

13. A pad assembly as claimed in any preceding claim, wherein the outlet tube (1) extends along the full length of the pad.

14. A pad assembly as claimed in any preceding claim, wherein the exterior (2) is conformable.

15. A urine collection device including a pad assembly as claimed in any preceding claim.

## Patentansprüche

1. Pad-Anordnung zum Gebrauch als eine nicht-invasive Schnittstelle einer Urinsammelvorrichtung, mit:
einer für Flüssigkeit undurchlässigen äußeren Schicht (2) mit einer Urin aufnehmenden Öffnung, einem oberen Ende und einem unteren Ende (4),
einer Schicht aus flexiblem Füllmaterial (3), durch das Urin im Wesentlichen frei fließen kann, wobei das Füllmaterial teilweise von der äußeren Schicht umgeben ist; und
einem Auslassrohr (1) zur Verwendung beim Abzug von Urin aus der Pad-Anordnung,
**dadurch gekennzeichnet, dass** sich das Auslassrohr (1) durch die äußere Schicht (2) zum unteren Ende (4) der Pad-Anordnung erstreckt.

2. Pad-Anordnung nach Anspruch 1, wobei das Füllmaterial (3) eine einzelne Schicht aufweist.

3. Pad-Anordnung nach Anspruch 1 oder 2, wobei das Füllmaterial (3) es dem Urin erlaubt, mit einer Rate von wenigstens 25 ml/Sekunde hindurchzufließen.

4. Pad-Anordnung nach Anspruch 3, wobei das Füllmaterial (3) es dem Urin erlaubt, mit einer Rate von wenigstens 35 ml/Sekunde hindurchzufließen.

5. Pad-Anordnung nach einem vorhergehenden Anspruch, wobei das Füllmaterial (3) ein offenes Material geringer Dichte aufweist.

6. Pad-Anordnung nach einem vorhergehenden Anspruch, wobei das Füllmaterial (3) eine Dichte von 0,03 - 0,1 g/cm³ hat.

7. Pad-Anordnung nach einem vorhergehenden Anspruch, wobei die Porosität des Füllmaterials (3) zwischen 50 % und 99 % seines Volumens beträgt.

8. Pad-Anordnung nach einem vorhergehenden Anspruch, wobei das Füllmaterial (3) retikulierten Schaum, eine nichtgewebte Kunststofffaser oder ein Abstandsgewebe aufweist.

9. Pad-Anordnung nach einem vorhergehenden Anspruch, wobei die Pad-Anordnung in der Lage ist, ein Urinauslassrohr (1) innerhalb des Füllmaterials (3) aufzunehmen.

10. Pad-Anordnung nach einem vorhergehenden Anspruch, wobei das innere des Pads in der Lage ist, einen Endabschnitt eines Urinauslassrohrs (1) so aufzunehmen, dass bei Gebrauch wenigstens ein Abschnitt eines Urinauslassrohrs in Längsrichtung innerhalb der Umrandung des Äußeren aufgenommen ist.

11. Pad-Anordnung nach einem vorhergehenden Anspruch, wobei das untere Ende (4) der Pad-Anordnung enger als sein oberes Ende ist.

12. Pad-Anordnung nach einem vorhergehenden Anspruch, wobei das untere Ende der Pad-Anordnung abgestumpft oder offen ist und im Wesentlichen dieselbe Größe wie der Durchmesser des Auslassrohres (1) hat.

13. Pad-Anordnung nach einem vorhergehenden Anspruch, wobei das Auslassrohr (1) sich entlang der gesamten Länge des Pads erstreckt.

14. Pad-Anordnung nach einem vorhergehenden Anspruch, wobei das Äußere (2) anpassbar oder fügsam ist.

15. Urinsammeleinrichtung mit einer Pad-Anordnung nach einem vorhergehenden Anspruch.

## Revendications

1. Un agencement d'un tampon pour usage en tant qu'interface non invasive d'un dispositif de recueil d'urine, comprenant :
- une couche (2) externe imperméable aux liquides comprenant une ouverture de recueil des urines, une extrémité supérieure et une extrémité (4) inférieure ;
- une couche de matériau (3) de remplissage, flexible à travers lequel l'urine est capable de s'écouler substantiellement librement, le matériau de remplissage étant partiellement entouré par la couche externe, et
- un tube (1) d'évacuation pour assurer le retrait de l'urine du tampon, **caractérisé en ce que** le tube (1) d'évacuation se prolonge à travers la couche (2) externe jusqu'à l'extrémité (4) inférieure du tampon.

2. Un agencement d'un tampon selon la revendication 1, dans lequel le matériau (3) de remplissage comporte une couche unique.

3. Un agencement de tampon selon la revendication 1 ou 2, dans lequel le matériau (3) de remplissage permet à l'urine de s'écouler à une vitesse de au moins 25 ml/s.

4. Un agencement de tampon selon la revendication 3, dans lequel le matériau (3) de remplissage permet à l'urine de s'écouler à une vitesse de au moins 35 ml/s.

5. Un agencement de tampon selon l'une quelconque des revendications précédentes, dans lequel le matériau (3) de remplissage comprend un matériau ouvert, de faible densité.

6. Un agencement d'un tampon selon l'une quelconque des revendications précédentes, dans lequel le matériau (3) de remplissage a une densité de 0,03-0,1 g/cm³.

7. Un agencement d'un tampon selon l'une quelconque des revendications précédentes, dans lequel la porosité du matériau (3) de remplissage est comprise entre 50 et 99% de son volume.

8. Un agencement d'un tampon selon l'une quelconque des revendications précédentes, dans lequel le matériau (3) comprend une mousse réticulée, un non-tissé en fibres de matière plastique ou un textile entretoise tissé.

9. Un agencement d'un tampon selon l'une quelconque des revendications précédentes, dans lequel le tampon peut recevoir un tube (1) d'évacuation d'urine dans le matériau (3) de remplissage.

10. Un agencement d'un tampon selon l'une quelconque des revendications précédentes, dans lequel l'intérieur du tampon peut recevoir une extrémité d'un tube (1) d'évacuation tel que, en utilisation, au moins une partie du tube d'évacuation d'urine est logé longitudinalement dans le bord de la partie externe.

11. Un agencement d'un tampon selon l'une quelconque des revendications précédentes, dans lequel l'extrémité (4) inférieure du tampon est plus étroite que l'extrémité supérieure.

12. Un agencement d'un tampon selon l'une quelconque des revendications précédentes, dans lequel l'extrémité inférieure du tampon est épointée et substantiellement de même dimension que le diamètre du tube (1) d'évacuation.

13. Un agencement d'un tampon selon l'une quelconque des revendications précédentes, dans lequel le tube (1) d'évacuation se prolonge sur toute la longueur du tampon.

14. Un agencement d'un tampon selon l'une quelconque des revendications précédentes, dans lequel la partie externe (2) est conformable.

15. Un dispositif de recueil d'urine comprenant un agencement d'un tampon selon l'une quelconque des revendications précédentes.
